# EUROPEAN PATENT APPLICATION

(11) **EP 2 835 094 A1**
(43) Date of publication of application: **11.02.2015**
(21) Application number: 13773042.0
(22) Date of filing: 27.03.2013
(51) Int. Cl.: A61B 1/00, A61B 10/00, G01N 21/64

(54) **DEVICE FOR DETERMINING METASTASIS OF CANCER TO SENTINEL LYMPH NODE**

(30) Priority: 05.04.2012 JP 2012086546
(71) Applicant: SBI Pharmaceuticals Co., Ltd., Tokyo 106-6020 (JP)
(72) Inventor: INOUE, Katsushi, Tokyo 106-6020 (JP); TANAKA, Tohru, Tokyo 106-6020 (JP); ISHII, Takuya, Tokyo 106-6020 (JP); NAKAJIMA, Motowo, Tokyo 106-6020 (JP)
(74) Representative: Harding, Charles Thomas
(86) International application number: PCT/JP2013/002084
(87) International publication number: WO 2013/150745

(57) **Abstract**

Provided is a device capable of determining whether or not cancer has metastasized to a sentinel lymph node, such as the axillary lymph node, thickly covered with fat, safely and in a simple manner without confirmation by biopsy or extirpation. Fluorescence generated in a sentinel lymph node upon the excitation of protoporphyrin (a photosensitizing substance) derived from 5-ALA administered to a subject can be detected by passing a hollow tube, into which a small-diameter optical fiber for launching light and the small-diameter optical fiber for receiving light are inserted, through the lymph vessel to identify the presence or absence of the metastasis of cancer cells even before the surgery of cancer or during prognosis.

## Description

### Technical Field

The present invention relates to a device for determining the presence or absence of cancer cell metastasis in a sentinel lymph node, and more specifically to a device for detecting/identifying cancer metastasis to a sentinel lymph node by detecting fluorescence occurring when a photosensitizing substance, such as protoporphyrin IX (hereinafter also referred to as "PpIX"), accumulated in the sentinel lymph node of a subject is excited.

Cancer (malignant tumor) is the biggest cause of death in Japan, and is said to be experienced by 1 of 2 Japanese people. Cancer is also highly ranked as the cause of death in other developed countries. Thus, it is an earnest desire for people around the world, including Japanese people, to develop an effective therapy for cancer. Reportedly, breast cancer is currently top of all cancers experienced by females and has an incidence of 1 in 23 females.

Examples of currently performed therapeutic methods for cancer can include surgical therapy, radiotherapy, chemotherapy, and immunotherapy; for extirpative surgery for cancer as a surgical therapy, it has been a previous standard operative procedure to remove a sentinel lymph node considered to be susceptible to cancer metastasis.

The sentinel lymph node is a lymph node at which cancer cells leaving the primary lesion first arrive, and for example, breast cancer is believed to metastasize to the whole body after passing through the sentinel lymph node (particularly, the axillary lymph node) around the breast. Thus, "axillary lymph node dissection" for removing all of tumor and this axillary lymph node during surgery has been performed by endoscopically injecting a radioisotope-labeled substance or a dye such as indocyanine green as a tracer flowing into, and accumulating in, the lymph node to identify the axillary lymph node. However, the dissection of the lymph node cannot completely prevent recurrence when metastasis to other organs has already occurred, and for early breast cancer, steps to remove the lymph node actually free of metastasis are performed and have often resulted in decreased QOL of a patient receiving the axillary lymph node dissection, such as the patient's trouble with a secondary disease, for example, lymphatic edema.

It has been proved, in recent years, that because the number of cancer cells arriving at a sentinel lymph node is initially limited to one or several, the presence of only one to several cancer metastases in a sentinel lymph node examined may be considered as showing almost no metastasis to the lymph node therebeyond. Thus, a step such as the omission of a lymph node dissection or the reduction of the range of the removal of the lymph node has been able to be taken when cancer metastasis is determined to be absent by locally injecting a tracer near breast cancer before breast cancer surgery and performing the biopsy (rapid diagnosis during operation) of the sentinel lymph node during the surgery.

In order to diagnose cancer and confirm the presence or absence of recurrence, non-invasive diagnostic imaging is commonly used. However, for example, for mammography, the lymph node also often does not come within the scope of photographing therein. Metastasis can be determined in a case where extra-nodal invasion, the same pleomorphic calcification and the like in the node as those in the main lesion are observed; however, the accuracy of the determination is known to be not high for the present because the frequency of the case is extremely low and the sensitivity thereof is low. For ultrasonography, the use of a probe of at least 7.5 MHz, typically 10 MHz or more is considered to result in the accuracy thereof being more than 90%. However, the basis is reported to be not yet sufficient for enabling the determination that the evaluation of the axillary lymph node by ultrasonography or CT is effective (see, for example, Non-patent Document 1), and this evaluation is considered to practically owe a great deal to the skill of an examiner.

Photodynamic diagnosis (hereinafter also referred to as "PDD") has been developed as a diagnosis method for identifying the presence or absence of a disease and an affected area by accumulating a photosensitizing substance in a diseased tissue, causing the substance to emit fluorescence by irradiation with excitation light, and observing the fluorescence. For example, 5-aminolevulinic acid (hereinafter also referred to as "5-ALA") is known as an intermediate in a pathway of pigment biosynthesis broadly present in animals, plants, and fungi, and is typically biosynthesized from succinyl-CoA and glycine by 5-ALA synthetase. Although it has no photosensitivity per se, 5-ALA is known to be metabolized to PpIX by a series of enzymes of the heme biosynthetic pathway in cells and accumulated in tumor tissue and neovascular vessels. Because PpIX emits red fluorescence having a peak around 636 nm when receiving excitation light having a wavelength around 410 nm, 5-ALA can be used for the diagnosis of tumor by PDD. When irradiation with light having a wavelength of 600 to 700 nm is performed after directly accumulating PpIX in tumor tissue and neovascular vessels, singlet oxygen generated by the excitation of light is considered to be capable of degenerating/necrotizing cells; the development of photodynamic therapy (hereinafter also referred to as "PDT") for treating a target area has also been progressing.

Specifically have been developed an endoscope for PDT for performing treatment by irradiating with laser light for PDT the tumor site of a subject in which an oncotropic photosensitive substance is accumulated (see, for example, Patent Document 1); a sentinel lymph node detection system comprising a light source for generating white light containing excitation light, a first filter means placed between the light source and a photographic subject, for transmitting excitation light contained in the white light emitted from the light source, an imaging means for imaging the fluorescent image of the photographic subject obtained with the excitation light and the background light image of the photographic subject obtained with light of a portion of the wavelength band of the excitation light, a second filter means placed between the photographic subject and the imaging means, for allowing the fluorescent image and the background light image to pass therethrough, an image processing means for outputting the fluorescent image and the background light image in the form of a fluorescence image by image processing, and an image display means for displaying the fluorescence image (see, for example, Patent Document 2); an evaluation device for irradiating with the excitation light from the tip portion of a laser catheter a tissue by which a photosensitive agent absorbing the excitation light to emit fluorescence has been taken up, comprising a connection to which the laser catheter is removably attached, a light source for outputting the excitation light to the laser catheter through the connection, and a detecting section detecting the intensity of the fluorescence entering from the laser catheter through the connection, for evaluating the change of the tissue by the reaction of the excitation light emitted from the tip portion of the laser catheter with the photosensitive agent taken up by the tissue (see, for example, Patent Document 3); and the like.

In addition, it has been realized to capture fluorescence in a metastasized lymph node before extirpation in an animal experiment using mice (see, for example, Non-patent Document 2). However, because the main factor inhibiting the capability of deep tissue penetration of excitation light in PDD is considered to be fat, it has been thought that when the human lymph node covered by thick fat is targeted for observation, the study results in mice cannot be directly applied and it is difficult to apply the device/system used by insertion into a body cavity without highly invasive treatment such as stripping the fat layer.

### Prior Art Documents

### Patent Documents

Patent Document 1: Japanese unexamined Patent Application Publication No. 2008-86680
Patent Document 2: Japanese unexamined Patent Application Publication No. 2006-340796
Patent Document 3: Japanese unexamined Patent Application Publication No. 2011-212423

### Non-Patent Documents

Non-patent Document 1: Kagakuteki Konkyo ni Motozuku Nyugan Shinryo Gaidorain (Guideline for Medical Care of Breast Cancer Based on Scientific Basis) (4) Medical Examination/Diagnosis, ed. the Japanese Breast Cancer Society, 2005, Kanehara & Co., Ltd.
Non-patent Document 2: Int. J. Cancer: 125, 2256-2263 (2009)

### Summary of the Invention

### Object to be Solved by the Invention

An object of the present invention is to provide a device capable of identifying whether or not cancer has metastasized to a sentinel lymph node, such as an axillary lymph node, thickly covered with fat, safely and in a simple manner without biopsy or extirpation before cancer surgery or during prognosis.

### Means to Solve the Object

The present inventors have found that tumor in which PpIX is accumulated can be detected and identified in a sentinel lymph node for which cancer metastasis has conventionally been confirmed by biopsy/extirpation, without performing such a highly invasive step by administering a tumor detection agent to a subject, passing a hollow tube into which a small-diameter optical fiber is inserted through an afferent lymph vessel to an axillary lymph node to place the light emission end of the small-diameter optical fiber in the neighboring region of the axillary lymph node, and sounding a notification sound when fluorescence generated from PpIX derived from the tumor detection agent by launching excitation light to the axillary lymph node is detected. In addition, it has been found that if necessary, it may be a device capable of performing treatment using PDT by launching red light.

Thus, the present invention relates to [1] a device for irradiating with excitation light a photosensitizing substance accumulated in a sentinel lymph node of a subject and identifying the presence or absence of cancer cell metastasis in the sentinel lymph node around tumor based on the detected amount of fluorescence generated from the photosensitizing substance, comprising a light source section for outputting excitation light; a measuring section for measuring the detected amount of fluorescence generated from the photosensitizing substance; a small-diameter optical fiber for launching light, that is optically connected to the light source section at the light-output end to guide the excitation light output from the light source section to the neighboring region of the sentinel lymph node to launch the excitation light from the launching end to the sentinel lymph node; a small-diameter optical fiber for receiving light, that is optically connected to the measuring section at a light-input end, to receive from a light-receiving end light generated from the sentinel lymph node by the launching of the excitation light and to guide the light to the measuring section; a flexible hollow tube into which the small-diameter optical fiber for launching light and the small-diameter optical fiber for receiving light are inserted, having an outer diameter capable of passing through a lymph vessel; and an excitation light-cutting means for cutting off lights of wavelengths in a predetermined band of the excitation light, placed on the optical path from the light-receiving end to measuring equipment.

The present invention also relates to [2] a device for irradiating with excitation light a photosensitizing substance accumulated in a sentinel lymph node of a subject and identifying the presence or absence of cancer cell metastasis in the sentinel lymph node around tumor based on the detected amount of fluorescence generated from the photosensitizing substance, comprising a light source section for outputting excitation light; a measuring section for measuring the detected amount of fluorescence generated from the photosensitizing substance; a small-diameter optical fiber for launching-cum-receiving light, that is optically connected to the light source section at the light-output end to guide the excitation light output from the light source section to the neighboring region of the sentinel lymph node to launch the excitation light from the light-launching-cum-receiving end to the sentinel lymph node as well as receiving light generated from the sentinel lymph node by the launching of the excitation light from the light-launching-cum-receiving end to guide the light to the common port; a flexible hollow narrow tube into which the small-diameter optical fiber for launching-cum-receiving light is inserted, having an outer diameter capable of passing through a lymph vessel; and an excitation light-cutting means for cutting off lights of wavelengths in a predetermined band of the excitation light, placed on the optical path from the light-receiving end to measuring equipment, and [3] a device for irradiating with excitation light a photosensitizing substance accumulated in a sentinel lymph node of a subject and identifying the presence or absence of cancer cell metastasis in the sentinel lymph node around tumor based on a detected amount of fluorescence generated from the photosensitizing substance, comprising a light source section for outputting excitation light; a measuring section for measuring the detected amount of fluorescence generated from the photosensitizing substance; an optical fiber for outputting light, that is optically connected to the light source section at the light-output end to guide the excitation light output from the light source section to a reflection port in a light mixing/separating 3-port module; a band reflection/transmission mirror in the light mixing/separating 3-port module, for outputting the excitation light output from the reflection port to a common port by reflection as well as transmitting fluorescence entering from the common port; a small-diameter optical fiber for launching-cum-receiving light, for guiding the excitation light output from the common port to the neighboring region of the sentinel lymph node to launch the excitation light from the light-launching-cum-receiving end to the sentinel lymph node and to receive light generated from the sentinel lymph node by the launching of the excitation light from the light-launching-cum-receiving end to guide the light to the common port; an optical fiber for light input, that is optically connected to the measuring section at the light-input end, to guide the fluorescence passing through the band reflection/transmission mirror to the measuring section through a transmission port in the light mixing/separating 3-port module; and a flexible hollow narrow tube into which the small-diameter optical fiber for launching-cum-receiving light is inserted, having an outer diameter capable of passing through a lymph vessel.

In addition, the present invention relates to [4] the device according to any one of [1] to [3] above, wherein a tumor detection agent is 5-aminolevulinic acid (5-ALA), [5] the device according to any one of [1] to [4] above, further comprising a means for dispersing fluorescence and further comprising a sound announcement means for outputting a detection sound to inform when the detected amount of fluorescence generated from the photosensitizing substance is equal to or higher than a set value, [6] the device according to any one of [1] to [5] above, wherein the device can output light of a wavelength of 625 nm to 640 nm from the light source section when the detected amount of fluorescence generated from the photosensitizing substance is in the range of the set value, [7] the device according to any one of [1] to [6] above, wherein the measuring section detects fluorescence generated from the photosensitizing substance by imaging fluorescence generated from the sentinel lymph node, and [8] the diagnosis device according to any one of [1] to [7] above, wherein the photosensitizing substance is protoporphyrin IX (PpIX).

### Effect of the Invention

According to the device of the present invention, the presence or absence of cancer cell metastasis can be confirmed in a sentinel lymph node around which fat is thickly accumulated without performing biopsy or extirpation; PDT can be performed in that state, if necessary; and a significant alleviation in the distress, anxiety, stress, and the like of a patient associated with the histopathology of the sentinel lymph node is achieved because the device is low in invasiveness and has a structure ergonomically highly easy on patients compared to conventional apparatuses.

### Brief Description of Drawings

[Figure 1] Figure 1 is a schematic of a device for identifying the metastasis of cancer to a sentinel lymph node according to the present invention.
[Figure 2] Figure 2 is an enlarged view of a tip of a hollow tube into which a small-diameter optical fiber for launching light and a small-diameter fiber for receiving light are inserted.
[Figure 3] Figure 3 is a schematic of a device for identifying the metastasis of cancer to a sentinel lymph node, comprising a light mixing/separating 3-port module, according to the present invention.
[Figure 4] Figure 4 is an enlarged view of a tip of a hollow narrow tube into which a small-diameter optical fiber for launching-cum-receiving light is inserted.

### Mode of Carrying Out the Invention

The device of the present invention is not particularly limited provided that it is a device for irradiating with excitation light a photosensitizing substance accumulated in a sentinel lymph node of a subject and identifying the presence or absence of cancer cell metastasis in the sentinel lymph node around tumor based on the detected amount of fluorescence generated from the photosensitizing substance, comprising a light source section for outputting excitation light; a measuring section for measuring the detected amount of fluorescence generated from the photosensitizing substance; a small-diameter optical fiber for launching light, that is optically connected to the light source at the light-output end to guide the excitation light output from the light source section to the neighboring region of the sentinel lymph node to launch the excitation light from the launching end to the sentinel lymph node; a small-diameter optical fiber for receiving light, that is optically connected to the measuring section at the light-input end, to receive from the light-receiving end reflected light and fluorescence generated from the sentinel lymph node by the launching of the excitation light and to guide the light to the measuring section; a flexible hollow tube into which the small-diameter optical fiber for launching light and the small-diameter optical fiber for receiving light are inserted, having an outer diameter capable of passing through a lymph vessel; and an excitation light-cutting means for cutting off lights of wavelengths in a predetermined band of the excitation light, placed on the optical path from the light-receiving end to measuring equipment [hereinafter sometimes referred to as "device I (1)"], a device for irradiating with excitation light a photosensitizing substance accumulated in a sentinel lymph node of a subject and identifying the presence or absence of cancer cell metastasis in the sentinel lymph node around tumor based on the detected amount of fluorescence generated from the photosensitizing substance, comprising a light source section for outputting excitation light; a measuring section for measuring the detected amount of fluorescence generated from the photosensitizing substance; a small-diameter optical fiber for launching-cum-receiving light, that is optically connected to the light source section at the light-output end to guide the excitation light output from the light source section to the neighboring region of the sentinel lymph node to launch the excitation light from the light-launching-cum-receiving end to the sentinel lymph node as well as receiving light generated from the sentinel lymph node by the launching of the excitation light from the light-launching-cum-receiving end to guide the light to the common port; a flexible hollow narrow tube into which the small-diameter optical fiber for launching-cum-receiving light is inserted, having an outer diameter capable of passing through a lymph vessel; and an excitation light-cutting means for cutting off lights of wavelengths in a predetermined band of the excitation light, placed on the optical path from the light-receiving end to measuring equipment [hereinafter sometimes referred to as "device I (2)"], or a device for irradiating with excitation light a photosensitizing substance accumulated in a sentinel lymph node of a subject and identifying the presence or absence of cancer cell metastasis in the sentinel lymph node around tumor based on the detected amount of fluorescence generated from the photosensitizing substance, comprising a light source section for outputting excitation light; a measuring section for measuring the detected amount of fluorescence generated from the photosensitizing substance; an optical fiber for outputting light, that is optically connected to the light source section at the light-output end to guide the excitation light output from the light source section to a reflection port in a light mixing/separating 3-port module; a band reflection/transmission mirror in the light mixing/separating 3-port module, for outputting the excitation light output from the reflection port to a common port by reflection as well as transmitting fluorescence entering from the common port; a small-diameter optical fiber for launching-cum-receiving light, for guiding the excitation light output from the common port to the neighboring region of the sentinel lymph node to launch the excitation light from the light-launching-cum-receiving end to the sentinel lymph node and to receive light generated from the sentinel lymph node by the launching of the excitation light from the light-launching-cum-receiving end to guide the light to the common port; an optical fiber for light input, that is optically connected to the measuring section at the light-input end, to guide the fluorescence passing through the band reflection/transmission mirror to the measuring section through a transmission port in the light mixing/separating 3-port module; and a flexible hollow narrow tube into which the small-diameter optical fiber for launching-cum-receiving light is inserted, having an outer diameter capable of passing through a lymph vessel [hereinafter sometimes referred to as "device II"]. Examples of the subject can include mammals such as humans, monkeys, dogs, and cats.

Examples of the tumor can include cancers and tumors produced by the malignant alteration of epithelial cells and the like, such as skin cancer, lung cancer, tracheal and bronchial cancers, oral epithelial cancer, esophageal cancer, gastric cancer, colon cancer, rectal cancer, large bowel cancer, liver and intrahepatic bile duct cancers, kidney cancer, pancreas cancer, prostate cancer, breast cancer, uterine cancer, ovarian cancer, and brain tumor.

The sentinel lymph node around tumor is a lymph node at which cancer cells from tumor (primary lesion) first arrive through a lymph stream in a lymph vessel, and preferred examples thereof can include an axillary lymph node as the major sentinel lymph node for breast cancer. The use of the device of the present invention enables the diagnosis of the sentinel lymph node as well as the observation of the state of metastasis in a lymph node when the metastasis has advanced.

The photosensitizing substance used may be any of various substances, including amino acid derivatives, azo dye, xanthene derivatives, chlorin, tetrapyrrole derivatives, and phthalocyanine which have conventionally been known as photosensitizing substances; however, preferable examples can include porphyrin photosensitizing substances such as protoporphyrin, hematoporphyrin, hematoporphyrin derivatives, benzoporphyrin, and pentacarboxyporphyrin, and among others, particularly preferable examples can include PpIX.

To accumulate the above PpIX in a sentinel lymph node, a compound can be administered which is represented by general formula (I): R²R¹NCH₂COCH₂CH₂COR³ (wherein R¹ and R² each independently represent a hydrogen atom, an alkyl group having 1 to 24 carbons, an acyl group having 1 to 12 carbons, an alkoxycarbonyl group having 2 to 13 carbons, an aryl group having 6 to 16 carbons, or an aralkyl group having 7 to 22 carbons; and R³ represents a hydroxyl group, an alkoxy group having 1 to 24 carbons, an acyloxy group having 1 to 12 carbons, an alkoxycarbonyloxy group having 2 to 13 carbons, an aryloxy group having 6 to 16 carbons, an aralkyloxy group having 7 to 22 carbons, or an amino group), or a salt thereof (which are hereinafter sometimes collectively referred to as "ALAs"). Because ALAs are metabolized in as early as 24 hours, they have very little side effects such as solar photosensitivity; thus, ALAs are most suitable for PDD and PDT.

Examples of the alkyl group having 1 to 24 carbons in the formula (I) can include straight-chain or branched alkyl groups each having 1 to 24 carbons; preferred are alkyl groups each having 1 to 18 carbons, particularly preferably alkyl groups each having 1 to 6 carbons. Specific examples of the alkyl group having 1 to 6 carbons can include a methyl group, an ethyl group, a n-propyl group, an isopropyl group, a n-butyl group, a sec-butyl group, a t-butyl group, a n-pentyl group, and a n-hexyl group.

Examples of the acyl group having 1 to 12 carbons in the formula (I) can include straight-chain or branched alkanoyl groups each having 1 to 12 carbons, straight-chain or branched alkenylcarbonyl groups each having 3 to 12 carbons, monocyclic or polycyclic aroyl groups each having 5 to 12 carbons, and monocyclic or polycyclic aryloxycarbonyl groups each having 5 to 12 carbons. Particularly, alkanoyl groups each having 1 to 6 carbons are preferable; specific examples thereof can include a formyl group, an acetyl group, a propionyl group, a butyryl group, and a pentanoyl group. The number of carbons of the acyl group having 1 to 12 carbons includes that of a carbonyl carbon.

The alkoxycarbonyl group having 2 to 13 carbons in the formula (I) is preferably an alkoxycarbonyl group having 2 to 7 carbons; specific examples thereof can include a methoxycarbonyl group, an ethoxycarbonyl group, a n-propoxycarbonyl group, an isopropoxycarbonyl group, a butoxycarbonyl group, a pentyloxycarbonyl group, a hexyloxycarbonyl group, a heptyloxycarbonyl group, an octyloxycarbonyl group, a nonyloxycarbonyl group, a decyloxycarbonyl group, an undecyloxycarbonyl group, and a dodecyloxycarbonyl group. The number of carbons of the alkoxycarbonyl group having 2 to 13 carbons includes that of a carbonyl carbon.

Examples of the aryl group having 6 to 16 carbons in the formula (I) can include monocyclic or polycyclic aryl groups; specific examples thereof can include a phenyl group, a naphthyl group, an anthryl group, a phenanthryl group, and a pyrenyl group.

Examples of the aralkyl group having 7 to 22 carbons in the formula (I) can include the groups consisting of the above-described aryl groups each having 6 to 16 carbons and the above-described alkyl groups each having 1 to 6 carbons; specific examples thereof can include a benzyl group and a phenethyl group.

Examples of the alkoxy group having 1 to 24 carbons in the formula (I) can include straight-chain or branched alkoxy groups each having 1 to 24 carbons; preferred are alkoxy groups each having 1 to 16 carbons, particularly preferably alkoxy groups each having 1 to 12 carbons. Specific examples thereof can include a methoxy group, an ethoxy group, a n-propoxy group, an isopropoxy group, a n-butoxy group, a pentyloxy group, a hexyloxy group, an octyloxy group, a decyloxy group, and a dodecyloxy group.

Examples of the acyloxy group having 1 to 12 carbons in the formula (I) can include straight-chain or branched alkanoyloxy groups each having 1 to 12 carbons; preferred are alkanoyloxy groups each having 1 to 6 carbons. Specific examples thereof can include an acetoxy group, a propionyloxy group, a butyryloxy group, and a pentanoyloxy group.

The alkoxycarbonyloxy group having 2 to 13 carbons in the formula (I) is preferably an alkoxycarbonyloxy group having 2 to 7 carbons; specific examples thereof can include a methoxycarbonyloxy group, an ethoxycarbonyloxy group, a n-propoxycarbonyloxy group, an isopropoxycarbonyloxy group, a butoxycarbonyloxy group, a pentyloxycarbonyloxy group, and a hexyloxycarbonyloxy group. The number of carbons of the alkoxycarbonyloxy group having 2 to 13 carbons includes that of a carbonyl carbon.

Examples of the aryloxy group having 6 to 16 carbons in the formula (I) can include monocyclic or polycyclic aryloxy groups; specific examples thereof can include a phenoxy group, a naphthyloxy group, an anthryloxy group, a phenanthryloxy group, and a pyrenyloxy group. The aralkyloxy group having 7 to 22 carbons is preferably one having the above-described aralkyl group; specific examples thereof can include a benzyloxy group and a phenethyloxy group.

In the formula (I), R¹ and R² are each preferably a hydrogen atom. R³ is preferably a hydroxyl group, an alkoxy group, or an aralkyloxy group, more preferably a hydroxyl group or an alkoxy group having 1 to 12 carbons, particularly preferably a methoxy group or a hexyloxy group.

In the formula (I), the compound in which R¹ and R² are each a hydrogen atom and R³ is a hydroxyl group is 5-ALA which is a particularly preferable example. Preferred examples of the 5-ALA derivative other than 5-ALA can include 5-ALA esters such as 5-ALA methyl ester, 5-ALA ethyl ester, 5-ALA propyl ester, 5-ALA butyl ester, 5-ALA pentyl ester, and 5-ALA hexyl ester; particularly preferable examples thereof can include 5-ALA methyl ester and 5-ALA hexyl ester. It is disclosed, for example, in Japanese unexamined Patent Application Publication (Translation of PCT Application) No. 11-501914 that 5-ALA esters have the same physiological effect as that of 5-ALA.

Pharmacologically acceptable salts of 5-ALA or 5-ALA derivatives can include pharmacologically acceptable acid addition salts, metal salts, ammonium salts, and organic amine addition salts. Examples of the acid addition salt can include inorganic acid salts such as hydrochlorides, hydrobromates, hydroiodides, phosphates, nitrates, and sulfates; and organic acid addition salts such as formates, acetates, propionates, toluenesulfonates, succinates, oxalates, lactates, tartrates, glycolates, methanesulfonates, butyrates, valerates, citrates, fumarates, maleates, and malates. Examples of the metal salt can include alkali metal salts such as lithium salts, sodium salts, and potassium salts; alkaline earth metal salts such as magnesium salts and calcium salts; and salts of metals such as aluminum and zinc. Examples of the ammonium salt can include alkylammonium salts such as ammonium salts and tetramethylammonium salts. Examples of the organic amine salt can include salts such as triethylamine salts, piperidine salts, morpholine salts, and toluidine salts.

5-ALA or a 5-ALA derivative can be produced by any of the methods of chemical synthesis, microbe-based production, and enzyme-based production. For example, the acyl group for an amino group of a 5-ALA derivative or the ester group for a carboxyl group thereof can be produced by the acylation of the amino group, the esterification of the carboxyl group, or the like using an ordinary method of chemical synthesis. When it is desired to obtain a salt of 5-ALA or a 5-ALA derivative, a compound represented by the general formula (I) which is obtained in the form of a salt may be directly purified, and that which is obtained in free form may be dissolved or suspended in a suitable organic solvent, to which an acid or a base is then added for the formation of the salt by a common method. 5-ALA or a 5-ALA derivative may be also present in the form of an addition product with water or any of various solvents.

The dose of the tumor detection agent administered to a subject according to the present invention is not particularly limited provided that by using the device of the present invention, a photosensitizing substance derived from the tumor detection agent can be irradiated with excitation light to identify the presence or absence of cancer cell metastasis in a sentinel lymph node based on the detected amount of fluorescence generated from the photosensitizing substance; the oral dose when the photosensitizing substance or the like is ALAs can be, for example, 1 mg to 1,000 mg, preferably 5 mg to 100 mg, more preferably 10 mg to 30 mg, still more preferably 15 mg to 25 mg, per kg body weight in terms of 5-ALA. When ALAs are used in the form of a solution, to prevent the decomposition of the ALAs, the solution is preferably prepared by exercising care so that the aqueous solution does not become alkaline. When it becomes alkaline, the decomposition of the active ingredient can be prevented by removing oxygen.

The time from the administration of the tumor detection agent to the irradiation with excitation light is preferably the time period during which the photosensitizing substance is sufficiently accumulated in tumor tissue; specific examples thereof can include 2 to 12 hours, preferably 4 to 8 hours, when 5-ALA is administered.

The light source section for outputting excitation light used in the device I and device II of the present invention may be a well-known one; examples thereof can include violet LEDs and semiconductor lasers. However, to accurately identify the presence or absence of 1 to a plurality of cancer cells minutely scattered in a sentinel lymph node, a semiconductor laser source having a high irradiance and a narrow irradiation area is preferable and a mixed crystal semiconductor such as InGaN can be used as an element used in the light source.

As the excitation light can be selected light having a wavelength capable of inducing fluorescence having a peak around 636 nm characteristic of a photosensitizing substance. Light is preferably selected which has a peak capable of inducing fluorescence further having a second peak around 705 nm characteristic of a photosensitizing substance. More preferred is light having a violet wavelength close to ultraviolet light belonging to the absorption peak of a photosensitizing substance belonging to the so-called Soret band; specific examples thereof can include light having a wavelength having a peak at 410 ± 10 nm, preferably 410 ± 5 nm, more preferably 410 ± 2 nm. For example, the blending ratio of the above-described InGaN can be changed to perform irradiation with suitable excitation light.

The small-diameter optical fiber for launching light in the device I (1) of the present invention is optically connected to the light source section at the light-output end; the excitation light output from the light source section can be guided to the neighboring region of a sentinel lymph node to launch the excitation light from the launching end to the sentinel lymph node. Examples of the light generated from the sentinel lymph node by the launching of the excitation light can include reflected excitation light from the sentinel lymph node, autofluorescence having a longer wavelength than that of the excitation light, from an inherent fluorescent substance in the sentinel lymph node, and fluorescence produced by the excitation of the photosensitizing substance accumulated in cancer cells in the sentinel lymph node, derived from the tumor detection agent.

The small-diameter optical fiber for receiving light in the device I (1) of the present invention receives the light generated from the sentinel lymph node through the light-receiving end and guides the light to the measuring section; however, an excitation light-cutting means can be placed on the optical path of the small-diameter optical fiber for receiving light from the light-receiving end to measuring equipment to cut off lights of wavelengths in a predetermined band of the excitation light, enabling the detection of fluorescence without being affected by the excitation light in the measuring section.

The hollow tube in the device I (1) of the present invention is not particularly limited provided that it is a flexible hollow tube into which the small-diameter optical fiber for launching light and the small-diameter optical fiber for receiving light are inserted, having an outer diameter capable of passing through a lymph vessel. The insertion means that the tips of the small-diameter optical fiber for launching light and the small-diameter optical fiber for receiving light inserted through one end of the hollow tube arrive at the other end of the hollow tube. Preferably, the small-diameter optical fiber for launching light and the small-diameter optical fiber for receiving light are positively supported in an inserted state without any position gap. Examples of the manner of support in an inserted state in the hollow tube can include the case of support by simple insertion by adjusting the internal diameter of the hollow tube and the case of support by a fixation part provided in the hollow tube. Examples of the fixation part can include an arcuate fixed holder; however, the fixation may be performed using a suitable adhesive or the like.

Specific examples of the excitation light-cutting means in the device I (1) or I (2) of the present invention can include a band reflection/transmission mirror and a cut filter capable of reflecting light of shorter wavelengths around the excitation light and transmitting lights of wavelengths longer than the excitation light.

The optical fiber for outputting light in the device II of the present invention is optically connected to the light source section at the light-output end and guides the excitation light output from the light source section to the reflection port in the light mixing/separating 3-port module. The band reflection/transmission mirror is placed in the common port to output the reflected excitation light to the common port.

The band reflection/transmission mirror in the light mixing/separating 3-port module in the device II of the present invention reflects the excitation light output from the light source section to output the light to the common port, and cuts off lights of wavelengths in a predetermined band of the excitation light among lights generated from the sentinel lymph node receiving the light from the light-launching-cum-receiving end to transmit fluorescence.

The small-diameter optical fiber for launching-cum-receiving light in the device II of the present invention guides the excitation light output from the common port to the neighboring region of the sentinel lymph node to launch the excitation light to the sentinel lymph node through the light-launching-cum-receiving end and receives the light generated from the sentinel lymph node by the launching of the excitation light through the light-launching-cum-receiving end to guide the light to the common port.

The optical fiber for light input in the device II of the present invention is optically connected to the measuring section at the light-input end and guides the fluorescence guided to the transmission port to the measuring section. The band reflection/transmission mirror can be placed in the common port to cut off lights of wavelengths in a predetermined band of the excitation light, enabling the detection of the guided fluorescence without being affected by the excitation light in the measuring section.

The hollow narrow tube in the device II or I (2) of the present invention is not particularly limited provided that it is a flexible hollow narrow tube into which the small-diameter optical fiber for launching-cum-receiving light is inserted, having an outer diameter capable of passing through a lymph vessel. The insertion means that the tip of the small-diameter optical fiber for launching-cum-receiving light inserted through one end of the hollow narrow tube arrives at the other end of the narrow hollow tube. Preferably, the small-diameter optical fiber for launching-cum-receiving light is positively supported in an inserted state without any position gap. Examples of the manner of support in an inserted state in the hollow narrow tube can include the case of support by simple insertion by adjusting the internal diameter of the hollow narrow tube and the case of support by a fixation part provided in the hollow narrow tube. Examples of the fixation part can include an arcuate fixed holder; however, the fixation may be performed using a suitable adhesive or the like.

"The hollow tube and the hollow narrow tube being capable of passing through a lymph vessel" means that they can be inserted into, removed from, or left in, the lymph vessel and "the hollow tube and the hollow narrow tube being flexible" means that they can be bent. This specifically means that these tubes can be inserted into, removed from, or left in, the lymph vessel without damaging the lymph vessel wall even by the curve of the lymph vessel; examples of the outer diameter of the hollow tube or the hollow narrow tube capable of passing through the lymph vessel can include 0.1 to 1.0 mm, preferably 0.5 to 0.95 mm, 0.8 to 0.90 mm, and examples of the inner diameter of the hollow tube or the hollow narrow tube can include 0.05 to 0.95 mm, preferably 0.5 to 0.9 mm, 0.6 to 0.85 mm. Such a hollow tube or such a hollow narrow tube can pass through an afferent lymph vessel to the sentinel lymph node.

The longitudinal length of the hollow tube can be properly determined, relying on the length necessary for guiding excitation light to the neighboring region of the sentinel lymph node by passing through the afferent lymph vessel from the body surface; it is, for example, 30 cm to 2 m, preferably 50 cm to 1 m. The longitudinal length of the hollow narrow tube is 30 cm to 2 m, preferably 50 cm to 1 m. It is, for example, 20 cm to 1.5 m, preferably 40 cm to 80 cm.

The material of the hollow tube or the hollow narrow tube is not particularly limited provided that it is a material bringing about the flexibility in the hollow tube or the hollow narrow tube; examples of such hollow tubes or hollow narrow tubes can include those made of a metal such as stainless steel, a plastic such as polyurethane, silicone, polyvinyl chloride, polybutadiene, polyamide, and ethylene-vinyl acetate copolymer, silicone, latex, and latex rubber whose surface is coated with silicone, a hydrophilic material, or silver.

The launching end and the light-receiving end, or the light-launching-cum-receiving end preferably has a form in which the fiber is properly exposed from an end of the hollow tube or the hollow narrow tube so that light can be positively launched to the sentinel lymph node and received from the sentinel lymph node; examples of the length of the exposed fiber can include 0.1 to 1 mm, preferably 0.2 to 0.8 mm, more preferably 0.3 to 0.6 mm. Particularly in the device II, the small-diameter optical fiber for launching-cum-receiving light can also be constructed so that it is freely advanced and retracted in the hollow narrow tube. The adoption of the construction enables, for example, only the small-diameter optical fiber for launching-cum-receiving light to be penetrated into the sentinel lymph node while being controlled when the hollow narrow tube passes through the lymph vessel and arrives at the inlet of the sentinel lymph node. In this case, the light-launching-cum-receiving end of the small-diameter optical fiber for launching-cum-receiving light can be exposed 1 to 15 mm, preferably 2 to 10 mm, more preferably 3 to 8 mm from the hollow narrow tube to enter into the sentinel lymph node to more precisely irradiate the sentinel lymph node with excitation light to receive light generated from the sentinel lymph node.

The hollow tube and the hollow narrow tube are preferably freely attached and detached from the light source section and the measuring section and thereby can be more easily washed/sterilized and can also be properly exchanged according to the type of the fiber to be inserted. The attachment and detachment may be performed using an adaptor.

When the device I (1) of the present invention is used, the excitation light output from the light source section is guided by the small-diameter optical fiber for launching light optically connected to the light source section at the light-output end and launched from the launching end to the sentinel lymph node as a target. The light generated from the sentinel lymph node is received at the light-receiving end, guided to the small-diameter optical fiber for receiving light, and guided to the measuring section through the excitation light-cutting means for cutting off lights of wavelengths in a predetermined band of the excitation light, placed on the optical path from the light-receiving end to the measuring equipment. The small-diameter optical fiber for launching light and the small-diameter optical fiber for receiving light are inserted into the flexible hollow tube having an outer diameter capable of passing through the lymph vessel

When the device I (2) of the present invention is used, the excitation light output from the light source section is guided by the small-diameter optical fiber for launching-cum-receiving light optically connected to the light source section at the light-output end and launched from the light-launching-cum-receiving end to the sentinel lymph node as a target. The light generated from the sentinel lymph node is received at the light-launching-cum-receiving end, guided to the small-diameter optical fiber for launching-cum-receiving light, and guided to the measuring section through the excitation light-cutting means for cutting off lights of wavelengths in a predetermined band of the excitation light, placed on the optical path from the light-receiving end to the measuring equipment. The small-diameter optical fiber for launching-cum-receiving light is inserted into the flexible hollow narrow tube having an outer diameter capable of passing through the lymph vessel.

When the device II of the present invention is used, in the device comprising a module having the 3 ports of a reflection port to which the optical fiber for outputting light connected to the light source section at the light-output end is connected at the other end, a transmission port to which the optical fiber for light input, connected to the measuring section at the light-input end is connected at the other end, and a common port to which the small-diameter optical fiber for launching-cum-receiving light whose one end is a light-launching-cum-receiving end is connected at the other end, the excitation light output from the light source section arrives at the light mixing/separating 3-port module through the reflection port, is reflected by the band reflection/transmission mirror, is guided by the small-diameter optical fiber for launching-cum-receiving light through the common port, and is launched from the light-launching-cum-receiving end to the sentinel lymph node as a target. The light generated from the sentinel lymph node is received at the light-launching-cum-receiving end and passes through the band reflection/transmission mirror by way of the common port to cut the excitation light to guide fluorescence from the transmission port to the measuring equipment. The insertion of the small-diameter optical fiber for launching-cum-receiving light as integral fibers into the hollow narrow tube enables the inner diameter and outer diameter of the hollow narrow tube to be made smaller.

The devices I (1) and I (2) and device II of the present invention may also further comprise a spectroscopic means. The spectroscopic means is not particularly limited provided that it can detect the intensity of fluorescence at a wavelength of 636 ± 2 nm, preferably 636 ± 1 nm and a wavelength of 626 ± 2 nm, preferably 626 ± 1 nm in order to detect fluorescence from a photosensitizing substance; however, the means is more preferably one capable of detecting fluorescence at a wavelength of 670 ± 2 nm from a partially ring-opened photosensitizing substance because the precision of the confirmation of the presence of the photosensitizing substance is further increased. Specifically, a wavelength-dispersive spectroscope such as a grating spectroscope or a prism spectroscope can be used; such a spectroscopic means may be interposed on the optical path from the light-receiving end or the light-launching-cum-receiving end to the measuring section, or may be mounted on the measuring section.

The measuring section according to the present invention may comprise a signal means based on the results of detecting the optical spectra obtained by the spectroscopic detection means. Examples of the signal means can include a sound announcement means or a visual signal means, and examples of the sound announcement means can include a means for announcing a sound by outputting a detection notice sound. For example, the sound announcement means may also output a detection sound when the intensity at the wavelength of 636 ± 10 nm, preferably 636 ± 5 nm, more preferably 636 ± 2 nm is not less than a set value or when the intensity at the wavelength of 670 ± 2 nm is not less than a set value; however, it is preferably a sound announcement means outputting a detection notice sound when the intensity of fluorescence at the wavelengths of 636 ± 2 nm and 626 ± 2 nm is detected in the measuring equipment and the difference between the fluorescence intensities at the wavelengths of 636 ± 2 nm and 626 ± 2 nm is not less than a set value, to eliminate the influence of the wavelength of the autofluorescence generated from the sentinel lymph node to more accurately detect the peak of the photosensitizing substance.

The presence or absence of cancer cells as well as information such as the size and density of cancer tissue can be transmitted to practitioners by setting to change the loudness, period of interruption, and frequency of the detection sound. For example, (a) no detection sound is outputted from the portion of normal cells; (b) a detection sound having a normal volume and a relatively long interruption period can be outputted when metastasis is determined to be absent because the number of cancer cells is 1 to 7 and the difference between the intensities of fluorescence is in the range of a predetermined set value; and (c) a detection sound having a slightly large volume or a slightly short interruption period can be outputted when metastasis probably has occurred because the number of cancer cells is 8 or more and the difference between the intensities of fluorescence is not less than a predetermined set value.. By using the device with such setting of notification sounds, practitioners can determine whether or not to extirpate the sentinel lymph node before the extirpative surgery for cancer, and also can confirm the presence or absence of prognostic recurrence.

The light source section can further output light for photodynamic therapy (PDT) in cancer cells confirmed in the sentinel lymph node; specifically, the device can also comprise a light source capable of outputting light having a wavelength of 625 nm to 640 nm, preferably a wavelength of 636 nm. The light source outputting red light used may be a well-known one; examples thereof can include a red semiconductor laser, red LED, and a discharge lamp with a strong red emission spectrum. The red light is guided to the sentinel lymph node by the small-diameter optical fiber for launching light and optically excites oxygen molecules in the surroundings by exciting the photosensitizing substance specifically accumulated in cancer cells in the sentinel lymph node, and the ensuing singlet oxygen has a cytotoxic effect against the cancer cells due to its strong oxidation power. For patients in whom cancer metastasis is determined to remain in the sentinel lymph node, the use of the device of the present invention further reduces the burden of the patients by simultaneously performing PDT in continuation of confirmation of the presence or absence of cancer cells.

In another form, the measuring section according to the present invention may also comprise an image section for displaying a spectroscopic image based on the results of detecting the optical spectra obtained using the spectroscope in the spectroscopic detection means. For example, the optical signals guided by a small-diameter optical fiber for receiving light or an optical fiber for light input, doubling as an image guide fiber, are received by a CCD camera and read as image signals according to the photographic subject image in sequence to form the image of the sentinel lymph node. In addition, an almost colorless sharp cut filter can be mounted as an excitation light-cutting means and used to cut off reflected light having a short wavelength close to ultraviolet light, and cancer cells can be visually confirmed using red fluorescence from the photosensitizing substance.

The device of the present invention can be inserted into the breast duct, due to the shape thereof; thus, it can also be applied to ductal breast cancer for which PDT has not conventionally been able to be performed. It can also be expected to be applied to the approach of the tip of a very small diameter endoscope, telescope, or the like to the lymph node by puncture.

The embodiments of the present invention will be described below with reference to the drawings. However, the present invention is not intended to be limited thereto.

### [First Embodiment; Device I (1)]

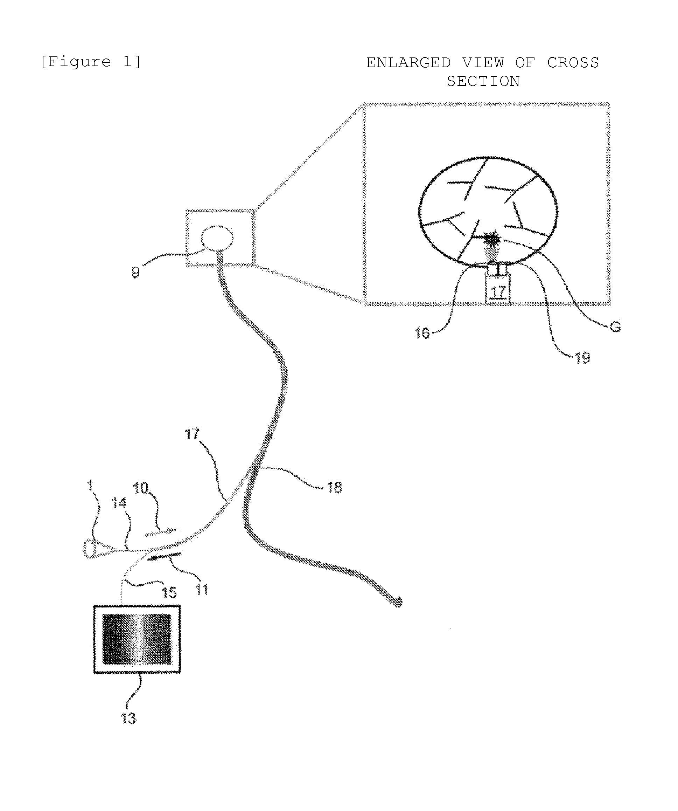

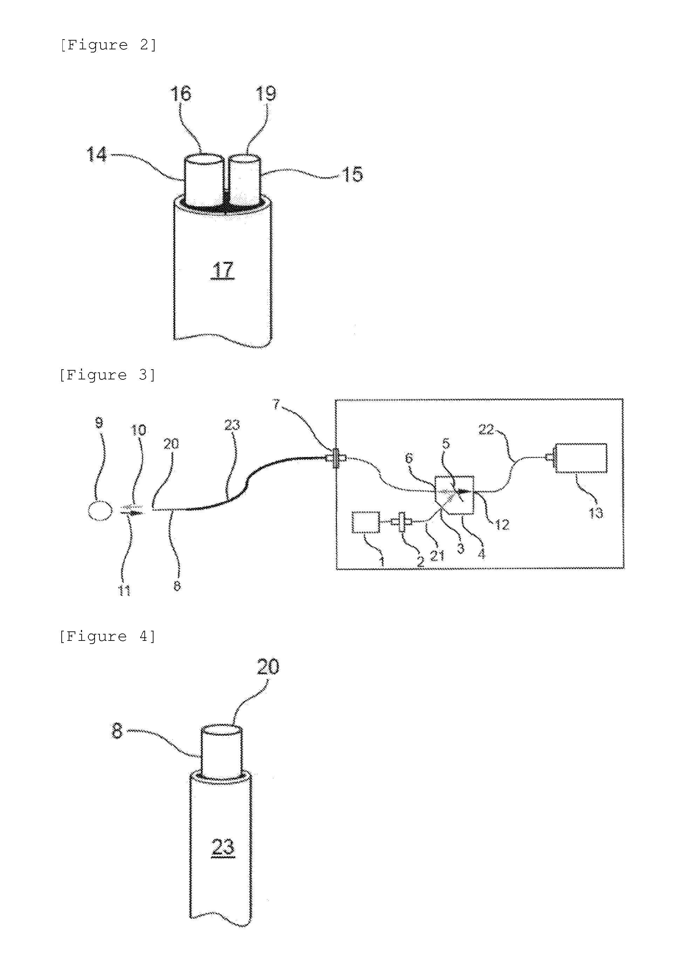

As shown in Figures 1 and 2, a hollow tube 17 into which a small-diameter optical fiber for launching light 14 and a small-diameter fiber for receiving light 15 are inserted passes through an afferent lymph vessel 18, and the small-diameter optical fiber for launching light 14 can guide excitation light 10 output from a light source section 1 to a launching end 16 to launch the excitation light from the launching end 16 to a sentinel lymph node 9. Reflected light, autofluorescence, and fluorescence from a photosensitizing substance 11 from cancer metastasis G in the sentinel lymph node 9 generated by irradiation with the excitation light are received by a light-receiving end 19 of the small-diameter optical fiber for receiving light 15 and enter the measuring section through a spectroscope 13 on which an excitation light cut filter is mounted.

### [Second Embodiment; Device II]

As shown in Figures 3 and 4, the excitation light output from the light source section 1 is optically connected to the light-output end of an optical fiber for outputting light 21 through a connection adaptor (1) 2. When the excitation light arrives at a light mixing/separating 3-port module 4 through a reflection port 3, the excitation light is reflected by a band reflection/transmission mirror 5; the reflected excitation light is guided to a small-diameter optical fiber for launching-cum-receiving light 8 inserted into a hollow narrow tube 23 passing through the lymph vessel through a common port 6 and a connection adaptor (2) 7; and the excitation light 10 is launched from a light-launching-cum-receiving end 20 towards the sentinel lymph node 9. The light 11 generated at the sentinel lymph node is received by the light-launching-cum-receiving end 20 and guided to the light mixing/separating 3-port module 4; the excitation light is reflected by the band reflection/transmission mirror 5; and fluorescence, which has a longer wavelength, passes therethrough and is guided to a measuring section 13 comprising a spectroscope by an optical fiber for light input 22 through a transmission port 12.

### Explanation of Letters or Numerals

- 1: Light Source Section
- 2: Connection Adaptor (1)
- 3: Reflection Port
- 4: Light Mixing/Separating 3-Port Module
- 5: Band Reflection/Transmission Mirror
- 6: Common Port
- 7: Connection Adaptor (2)
- 8: Small-Diameter Optical Fiber for Launching-Cum-Receiving Light
- 9: Sentinel Lymph Node
- 10: Excitation Light
- 11: Light Generated at Sentinel Lymph Node
- 12: Transmission Port
- 13: Measuring Section (Spectroscope)
- 14: Small-Diameter Optical Fiber for Launching Light
- 15: Small-Diameter Optical Fiber for Receiving Light
- 16: Launching End
- 17: Hollow Tube
- 18: Afferent Lymph Vessel
- 19: Light-Receiving End
- 20: Light-Launching-Cum-Receiving End
- 21: Optical Fiber for Outputting Light
- 22: Optical Fiber for Light Input
- 23: Hollow Narrow Tube
- G: Cancer Metastasis

## Claims

1. A device for irradiating with excitation light a photosensitizing substance accumulated in a sentinel lymph node of a subject and identifying the presence or absence of cancer cell metastasis in the sentinel lymph node around tumor based on a detected amount of fluorescence generated from the photosensitizing substance, comprising:
a light source section for outputting the excitation light;
a measuring section for measuring the detected amount of fluorescence generated from the photosensitizing substance;
a small-diameter optical fiber for launching light, that is optically connected to the light source section at a light-output end to guide the excitation light output from the light source section to a neighboring region of the sentinel lymph node to launch the excitation light from a launching end to the sentinel lymph node;
a small-diameter optical fiber for receiving light, that is optically connected to the measuring section at a light-input end, to receive from a light-receiving end light generated from the sentinel lymph node by the launching of the excitation light and to guide the light to the measuring section;
a flexible hollow tube into which the small-diameter optical fiber for launching light and the small-diameter optical fiber for receiving light are inserted, having an outer diameter capable of passing through a lymph vessel; and
an excitation light-cutting means for cutting off lights of wavelengths in a predetermined band of the excitation light, placed on an optical path from the light-receiving end to measuring equipment.

2. A device for irradiating with excitation light a photosensitizing substance accumulated in a sentinel lymph node of a subject and identifying the presence or absence of cancer cell metastasis in the sentinel lymph node around tumor based on a detected amount of fluorescence generated from the photosensitizing substance, comprising:
a light source section for outputting excitation light;
a measuring section for measuring the detected amount of fluorescence generated from the photosensitizing substance;
a small-diameter optical fiber for launching-cum-receiving light, that is optically connected to the light source section at a light-output end to guide the excitation light output from the light source section to a neighboring region of the sentinel lymph node to launch the excitation light from a light-launching-cum-receiving end to the sentinel lymph node as well as receiving light generated from the sentinel lymph node by the launching of the excitation light from the light-launching-cum-receiving end to guide the light to the common port;
a flexible hollow narrow tube into which the small-diameter optical fiber for launching-cum-receiving light is inserted, having an outer diameter capable of passing through a lymph vessel; and
an excitation light-cutting means for cutting off lights of wavelengths in a predetermined band of the excitation light, placed on an optical path from the light-receiving end to measuring equipment.

3. A device for irradiating with excitation light a photosensitizing substance accumulated in a sentinel lymph node of a subject and identifying the presence or absence of cancer cell metastasis in the sentinel lymph node around tumor based on a detected amount of fluorescence generated from the photosensitizing substance, comprising:
a light source section for outputting excitation light;
a measuring section for measuring the detected amount of fluorescence generated from the photosensitizing substance;
an optical fiber for outputting light, that is optically connected to the light source section at a light-output end to guide the excitation light output from the light source section to a reflection port in a light mixing/separating 3-port module;
a band reflection/transmission mirror in the light mixing/separating 3-port module, for outputting the excitation light output from the reflection port to a common port by reflection as well as transmitting fluorescence entering from the common port;
a small-diameter optical fiber for launching-cum-receiving light, for guiding the excitation light output from the common port to a neighboring region of the sentinel lymph node to launch the excitation light from a light-launching-cum-receiving end to the sentinel lymph node and to receive light generated from the sentinel lymph node by the launching of the excitation light from the light-launching-cum-receiving end to guide the light to the common port;
an optical fiber for light input, that is optically connected to the measuring section at a light-input end,
to guide the fluorescence passing through the band reflection/transmission mirror to the measuring section through a transmission port in the light mixing/separating 3-port module; and
a flexible hollow narrow tube into which the small-diameter optical fiber for launching-cum-receiving light is inserted, having an outer diameter capable of passing through a lymph vessel.

4. The device according to any one of claims 1 to 3, wherein a tumor detection agent is 5-aminolevulinic acid (5-ALA).

5. The device according to any one of claims 1 to 4, further comprising a means for dispersing fluorescence and further comprising a sound announcement means for outputting a detection sound to inform when the detected amount of fluorescence generated from the photosensitizing substance is equal to or higher than a set value.

6. The device according to any one of claims 1 to 5, wherein the device can output light of a wavelength of 625 nm to 640 nm from the light source section when the detected amount of fluorescence generated from the photosensitizing substance is in a range of the set value.

7. The device according to any one of claims 1 to 6, wherein the measuring section detects fluorescence generated from the photosensitizing substance by imaging fluorescence generated from the sentinel lymph node.

8. The diagnosis device according to any one of claims 1 to 7, wherein the photosensitizing substance is protoporphyrin IX (PpIX).
